# EUROPÄISCHE PATENTSCHRIFT

(11) **EP 2 313 073 B1**
(45) Veröffentlichungstag und Bekanntmachung des Hinweises auf die Patenterteilung: **06.12.2017**
(21) Anmeldenummer: 09780983.4
(22) Anmeldetag: 23.07.2009
(51) Int. Cl.: A61K 8/44, A61K 8/60, A61Q 5/02, A61Q 19/10

(54) **SULFATFREIES MILDES TENSIDSYSTEM ZUR HAUT- UND HAARREINIGUNG**
NON-SULFATE MILD SURFACTANT SYSTEM FOR SKIN AND HAIR CLEANING
SYSTÈME DE TENSIOACTIF DOUX SANS SULFATES POUR LE NETTOYAGE DE LA PEAU ET DES CHEVEUX

(30) Priorität: 18.08.2008 DE 102008038137
(43) Veröffentlichungstag der Anmeldung: 27.04.2011
(73) Patentinhaber: Henkel AG & Co. KGaA, 40589 Düsseldorf (DE)
(72) Erfinder: HIPPE, Thomas, 25482 Appen (DE); KURSAWE, Petra, 22527 Hamburg (DE)
(86) Internationale Anmeldenummer: PCT/EP2009/059495
(87) Internationale Veröffentlichungsnummer: WO 2010/020516

(56) Entgegenhaltungen:
- EP-A- 0 911 022
- EP-A- 1 672 056
- WO-A-03/028671
- CA-A1- 2 143 558
- DE-C1- 19 737 604
- US-A1- 2002 071 818
- US-A1- 2008 113 891
- "Cognis: Coming up with new concepts" FOCUS ON SURFACTANTS, ELSEVIER, Bd. 2005, Nr. 6, 1. Juni 2005 (2005-06-01), Seite 4, XP004974359 ISSN: 1351-4210

## Beschreibung

Die Erfindung betrifft sulfatfreie tensidhaltige Haut- und/oder Haarreinigungsmittel auf der Basis einer speziellen, milden Tensidmischung in einem bestimmten Verhältnis, die Verwendung dieser Tensidmischung zur Verbesserung der Hautverträglichkeit und zur Herstellung eines feinporigen, cremigen Schaums, sowie ein Verfahren zur Haut- und/oder Haarbehandlung unter Verwendung des Mittels.

Bedingt durch das stetig steigende Hygiene-Empfinden der Menschen und die damit immer größer und breiter werdenden Anforderungen an kosmetische Reinigungsmittel, ergeben sich fortwährend neue Problemstellungen und Schwierigkeiten bei deren Herstellung.

Um den Bedürfnissen der Verbraucher gerecht zu werden, müssen Reinigungsmittel, insbesondere kosmetische Reinigungsmittel für Haut und Haare, wie sie beispielsweise als flüssige Seifen, Shampoos, Duschbäder, Schaumbäder, Dusch- und Waschgele im Handel erhältlich sind, heutzutage nicht nur ein gutes Reinigungsvermögen aufweisen, sondern sollen weiterhin gut verträglich sein und auch bei häufiger Anwendung nicht zu starker Entfettung oder Trockenheit der Haut oder der Haare führen.

Übliche kosmetische Reinigungsprodukte enthalten meist anionische Tenside in Mengen von etwa 5 bis 20 Gew.-%, um eine zufriedenstellende Reinigungsleistung zu erzielen. Nach der Anwendung eines solchen Reinigungsprodukts empfindet der Verbraucher seinen Haut- und Haarzustand jedoch oftmals als nicht ideal, was vorwiegend auf die leicht irritierende Wirkung der meisten anionischen Tenside zurückzuführen ist. Der Hautzustand nach der Anwendung wird als trocken, gespannt und mitunter als rau empfunden, weshalb die nachträgliche Applikation von Cremes oder Lotionen erforderlich wird. Die Haare fühlen sich gleichsam trocken und spröde an, was sie anfällig für Haarbrüche und Spliss macht. Auch die Kämmbarkeit der Haare ist oft nicht zufriedenstellend, daher müssen auch die Haare nach der Reinigung mit Spülungen, Kuren und Tonics behandelt werden.

Dieser Nachbehandlungsschritt der Haut und/oder der Haare wird vom Verbraucher aber nicht immer gewünscht, geht doch damit sowohl ein beträchtlicher Zeit- als auch Kostenaufwand einher.

In der letzten Zeit hat es daher nicht an Versuchen gefehlt Mittel zur Verfügung zu stellen, die nicht nur eine zufriedenstellende Reinigung gewährleisten, sondern auch spürbare Pflegeeigenschaften auf der Haut oder den Haaren aufweisen.

So gelang es diverse Fettstoffe, Silikone und/oder kationische Polymere, kationische Tenside, spezielle Pflanzenextrakte etc. stabil in Reinigungszusammensetzungen einzuarbeiten und damit sogenannte "2in1"-Produkte zur Verfügung zu stellen, die den vorgenannten Anforderungen gerecht wurden.

Nachteilig an diesen Zusammensetzungen ist aber bis auf den heutigen Tag, dass sie nach wie vor auf anionischen (wenn auch milden) Sulfattensiden basieren. Der Grund dafür ist, dass der Verbraucher Shampoos und/oder Reinigungszusammensetzungen erwartet, die einen cremigen, feinporigen Schaum bilden, der sich angenehm anfühlt, und der sich leicht auf der Haut und den Haaren verteilen lässt.

Diese Anforderungen an den Schaum ließen sich bislang nur aber durch einen Gehalt an anionischen Sufattensiden erzielen, denn die Inkorporation der Pflegestoffe in die kosmetischen Reinigungsmittel, insbesondere die Inkorporation von Fettstoffen, wirkte sich oft negativ auf die Schaumqualität aus, was häufig sogar noch einen höheren Gehalt an anionischen Sulfattensiden und zusätzlich an Co-Tensiden und Schaumboostern erforderlich machte.

Neben "2in1"-Produkten, die möglichst sulfatfrei sind und einen cremigen, feinporigen Schaum bilden, verschiebt sich der Fokus bei der kosmetischen Reinigungsmittelherstellung in jüngster Zeit auch mehr und mehr in Richtung naturkosmetischer Produkte, die einen möglichst großen Anteil an Wirkstoffen natürlicher (pflanzlicher) Herkunft enthalten, welche nicht wesentlich chemisch (weiter)behandelt wurden und die optimalerweise biologisch abbaubar und ökologisch unbedenklich sind. Das steigende Umwelt - und Gesundheitsbewusstsein der Verbraucher hat dazu in entscheidendem Maße beigetragen.

Aufgabe der vorliegenden Erfindung war es daher ein kosmetisches Reinigungsmittel für die Haut und die Haare herzustellen, das den oben genannten Anforderungen gerecht wird.

Optimalerweise soll sowohl auf Sulfate, als auch auf PEG-Verbindungen in den Zusammensetzungen verzichtet werden, da es sich bei den PEG-Verbindungen oftmals um Stoffe natürlichen Ursprungs handelt, die chemisch behandelt wurden. Darüber hinaus neigen manche PEG-Verbindungen als Emulgator für Öl und Wasser dazu, die Haut für Schadstoffe durchlässiger zu machen, weshalb sie in kosmetischen Mitteln in Ökotest-Beurteilungen eher schlecht bewertet werden.

Daneben sollen die angestrebten Produkte neben einer effektiven Reinigungswirkung die innere Struktur der Keratinfasern stärken und damit die Geschmeidigkeit, den Griff, die Zugfestigkeit und die Kämmbarkeit von Keratinfasern verbessern.

Vollkommen überraschend wurde nun eine neue Tensidmischung gefunden, mit der die oben genannten Anforderungen in hohem Maße erfüllt werden.

Es gelang damit nicht nur kosmetische Reinigungsmittel herzustellen, die sulfat- und PEG-frei, sondern die auch sehr mild sind, und einen besonders cremigen, feinporigen und weichen Schaum bilden.

Des weiteren konnten Pflegestoffe stabil in die Rezepturen eingearbeitet werden, die eine nachhaltige Konditionierung der Haut und/oder der Haare bewirken und einen nachträglichen Behandlungsschritt mit Cremes, Salben, Haarkuren oder Spülungen entbehrlich machen.

Gegenstand der vorliegenden Erfindung sind damit Sulfat-freie Haut- und/oder Haarreinigungsmittel, die in einem kosmetisch akzeptablen Träger eine milde Tensidmischung aus
a. mindestens einem Acylaminosäuresalz wie in Anspruch 1 definiert,
b. mindestens einem Alkylpolyglucosid und
c. mindestens einem zwitterionischen und/oder amphoteren Tensid,
enthalten, wobei das Verhältnis der Komponenten (a) : (b) : (c) im Bereich von (0,1-1): (1-3): (0,1-5) liegt.

In einer bevorzugten Ausführungsform der Erfindung beträgt das Tensidverhältnis (a) : (b) : (c) (0,1-1) : (1-2): (0,5-3) und insbesondere 1 : (1-2): (0,5-2,5).

Neben sulfat-freien kosmetischen Haut- und/oder Haareinigungsmitteln sind erfindungsgemäß insbesondere auch solche Mittel bevorzugt, die auch keine PEG-Verbindungen enthalten. Unter "PEG-freien Verbindungen" werden erfindungsgemäß die folgenden Zusammensetzungen verstanden:
1) Verbindungen der Formel (I)

   **H-(O-CH₂CH₂)ₙ-OH** **(I),**

   in der n für ganze Zahlen zwischen 1 und 100.000 steht,
2) Verbindungen der Formel (II)

   **H-(O-CH(CH₃)CH₂)ₙ-OH** **(II),**

   in der n für ganze Zahlen zwischen 1 und 100.000 steht,
3) Verbindungen der Formel (III)

   **R-(O-CH₂CH₂)ₙ-OH** **(III),**

   in der R für einen Alkyl- oder Alkenylrest und n für Zahlen zwischen 1 und 10.000 steht,
4) Verbindungen der Formel (IV)

   **R-(O-CH₂CH₂)ₙ-OSO₃H** **(IV),**

   in der R für einen Alkyl- oder Alkenylrest und n für Zahlen zwischen 1 und 10.000 steht, und
5) Verbindungen der Formel (V)

   **-(O-CH₂CH₂)ₙ-** **(V),**

   in der n für ganze Zahlen zwischen 2 und 100.000 steht.

Die erfindungsgemäßen Mittel sind frei von Polyethylenglycolen der allgemeinen Formel (I), d.h. sie enthalten weder Ethylenglycol (n = 1) noch Produkte mit einem Polymeristaionsgrad Pₙ= 2-4 (Diethylenglycol, Triethylenglycol und Tetraethylenglycol) noch Polyethylenglycole mit höheren Polymerisationsgraden Pₙ von ca. 5 bis 100.000, die nicht mehr mol.-einheitlich herstellbar, sondern polydispers sind.

Sie sind bevorzugt auch frei von Polypropylenglycolen der allgemeinen Formel (II), d.h. sie enthalten weder Propylenglycol (n = 1) noch Produkte mit einem Polymeristaionsgrad Pₙ=2-4 (Dipropylenglycol, Tripropylenglycol und Tetrapropylenglycol) noch Polypropylenglycole mit höheren Polymerisationsgraden Pₙ von ca. 5 bis 100.000, die nicht mehr mol.-einheitlich herstellbar, sondern polydispers sind.

Es hat sich gezeigt, dass vorzugsweise auch auf den Einsatz ethoxylierter Verbindungen verzichtet werden sollte. Insbesondere nichtionische Tenside vom Typ der Alkyl- oder Alkenylethoxylate vermindern ebenfalls die positiven Effekte der erfindungsgemäßen Mittel und sollten deshalb in den Rezepturen nicht eingesetzt werden. Deshalb enthalten erfindungsgemäß bevorzugte Haut- und/oder Haarreinigungsmittel auch keine Verbindungen der Formel (IIII).

Da die erfindungsgemäßen Mittel zwingend sulfatfrei formuliert werden sollen, enthalten sie natürlich auch keine Alkylethersulfate der Formel (IV).

In besonders bevorzugten erfindungsgemäßen Mitteln wird gänzlich auf den Einsatz von Verbindungen verzichtet, die ethoxylierte Gruppierungen aufweisen.

Bevorzugte erfindungsgemäße Haut- und/oder Haarreinigungsmittel enthalten daher keine Verbindung, welche die Gruppierung **-(O-CH₂CH₂)ₙ-O-** mit n = 1 - 10.000 enthält. Besonders bevorzugte erfindungsgemäße Haut- und/oder Haarreinigungsmittel sind dadurch gekennzeichnet, dass sie keine Verbindungen der Formel (V) aufweisen.

Die erfindungsgemäße Tensidmischung wird in dem vorgenannten Verhältnis in den erfindungsgemäßen Haut- und/oder Haarreinigungsmitteln eingesetzt. Dabei entfallen auf die einzelnen Tensidbestandteile Mengen von
(a) 0,001 bis 15 Gew.-%, bevorzugt 0,005 bis 10 Gew.-%, mehr bevorzugt 0,01 bis 7,5 Gew.-% und insbesondere 0,05 bis 5 Gew.-%,
(b) 0,05 bis 15 Gew.-%, bevorzugt 0,1 bis 12,5 Gew.-%, mehr bevorzugt 0,5 bis 10 Gew.-% und insbesondere 1 bis 7,5 Gew.-%,
(c) 0,05 bis 20 Gew.-%, bevorzugt 0,1 bis 15 Gew.-%, mehr bevorzugt 0,5 bis 12,5 Gew.-% und insbesondere 1 bis 10 Gew.-%;
wobei die Mengenangaben sich jeweils auf das Gesamtgewicht der Haut- und/oder Haarreinigungsmittel beziehen.

Bei der anionischen Tensidkomponente (a) der erfindungsgemäßen Tensidkombination handelt es sich um ein Acylaminosäuresalz der Formel (VI), 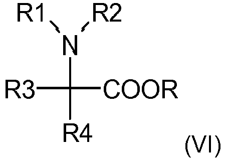 in der
- der Rest R für ein Wasserstoffatom, ein Alkali- oder Erdalkalimetallion, ein Ammonium Alkylammonium- oder Alkanolammoniumion steht,
- einer der Reste R1 oder R2 für Wasserstoff, eine Phenyl- oder eine -CH₂-COOR-Gruppe, und der andere Rest R1 oder R2 für eine COR'-Gruppen steht, in der R' für einen geradkettigen oder verzweigten, gesättigten oder ungesättigten Alkylrest mit 8 bis 30 C-Atomen steht,
- die Reste R3 und R4 unabhängig voneinander für Wasserstoff- oder lineare oder verzweigte Gruppen -CH(R5)-(CH₂)ₙ-COOR stehen, in denen R5 Wasserstoff oder eine C₁-C₄-Alkylgruppe ist und n für eine ganze Zahl von 0 bis 10 steht. Erfindungsgemäss bevorzugt sind die Acylaminosäuresalze der Aminosäuren Sarcosin, Asparaginsäure und Glutaminsäure und unter diesen insbesondere die Alkalisalze der Lauroylsarcosinate, Cocoylsarcosinate, Myristoylsarcosinate, Oleylsarcosinate, Lauroylglutamate, Cocoylglutamate, Myristoylglutamate und Stearoylglutamate, insbesondere die unter den Handelsnamen Protelan® MST 35, Perlastan® C 30, Hamposyl® O, Hamposyl® L 30, Hamposyl® L 95, Eumulgin® SG, Aminsoft® CS 11, Plantapon® ACG 35, Plantapon® ACG 50 und Protelan® AGL 95 vertriebenen Produkte, wobei es für einige Ausführungsformen der Erfindung besonders bevorzugt sein kann, wenn als Komponente (a) das unter der INCI-Bezeichnung Disodium Cocoyl Glutamate bekannte anionische Tensid eingesetzt wird.

Das zweite essentielle Tensid der erfindungsgemäßen Tensidmischung ist ein Alkylpoyglucosid.

Geeignete Alkylpolygykoside (APGs) entsprechend der allgemeinen Formel RO-(Z)ₓ wobei R für Alkyl, Z für einen Zucker sowie x für die Anzahl der Zuckereinheiten steht. Die erfindungsgemäßen APGs können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen. Besonders bevorzugt sind solche APGs, bei denen R im wesentlichen aus C₈- und C₁₀-Alkylgruppen, C₁₂- und C₁₄-Alkylgruppen, C₈- bis C₁₆-Alkylgruppen, C₁₂- bis C₁₆-Alkylgruppen oder C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Erfindungsgemäß besonders geeignete Alkylpolyglucoside sind die unter der INCI-Bezeichnung Lauryl Glucoside, Coco-Glucoside, Decyl Glucoside und C₈-C₁₀-Alkylpolyglucoside bekannten Produkte, die in den erfindungsgemäßen Zusammensetzungen auch als wässrige Lösung oder als wässrige Lösung in der Mischung mit anderen Tensiden eingesetzt werden können.

Als dritte essentielle Tensidkomponente der erfindungsgemäßen Tensidmischung wird ein amphoteres und/oder zwitterionisches Tensid eingesetzt, das ausgewählt ist aus der Gruppe der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionaten,
- Kokosacylaminoethylaminopropionaten
- C₁₂ - C₁₈ - Acylsarcosinen,
- N-Alkyl-N,N-dimethylammoniumglycinaten, z.B. Kokosalkyldimethylammoniumglycinat,
- N-Acyl-aminopropyl-N,N-dimethylammoniumglycinaten, beispielsweise Kokosacylaminopropyl-dimethylammoniumglycinat,
- 2-Alkyl-3-carboxymethyl-3-hydroxyethyl-imidazolinen mit jeweils 8 bis 18 C-Atomen in der Alkyl- oder Acylgruppe
- Kokosacylaminoethylhydroxyethylcarboxymethylglycinat
- den unter der INCI-Bezeichnung Cocamidopropyl Betain bekannten Verbindungen,
- den unter der INCI-Bezeichnung Disodium Cocoamphodiacetate bekannten Verbindungen,
- den unter der INCI-Bezeichnung Betaine bekannten Verbindungen.

Daneben ist ein weiteres insbesondere bevorzugtes zwitterionisches und/oder amphoteres Tensid eine Verbindung der Formel (IX) 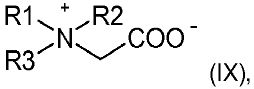 in der die Reste R1 bis R3 unabhängig voneinander für C₁-C₄-Alkylgruppen oder Hydroxyalkylgruppen stehen. Bevorzugt stehen die Reste R1 bis R3 für gleiche Reste Methyl- oder Ethyl und insbesondere bevorzugte stehen alle drei Reste R1, R2 und R3 für Methylreste.

Ein erfindungsgemäß besonders geeignetes Tensid (c) ist das unter der INCI-Bezeichnung "Betaine" vertriebene Produkt der Formel (IX), beispielsweise das unter dem Handelsnamen Tego Natural Betaine® vertriebene Tensid der Firma Goldschmidt (Evonik-Degussa). Dieses Tensid ist erfindungsgemäß insbesondere geeignet, weil es überwiegend natürlichen Ursprungs ist und neben seinen tensidischen Eigenschaften auch ausgezeichnete Moisturizing- und Hautpflegeeigenschaften aufweist.

In einer bevorzugten Ausführungsform der Erfindung enthalten die erfindungsgemäßen Haut- und/oder Haareinigungsmittel neben der milden Tensidmischung mindestens eine haut- und haarkonditionierende Komponente, die der folgenden Formel (X) entspricht: 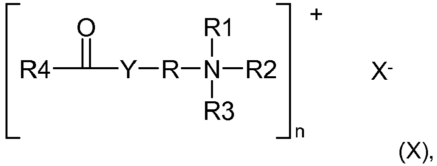 in der
- R4 für eine lange aliphatische Kohlenstoffgruppe mit 8 bis 24 C-Atomen steht,
- Y für ein Heteroatom, ausgewählt aus O, N oder S steht, wobei im Falle von Y=N das Stickstoffatom noch einen Substituenten -H oder -C₁-C₄-Alkyl trägt,
- R für eine geradekettige oder verzweigte Alkylengruppe mit 1 bis 10 C-Atomen steht,
- R1, R2 und R3 unabhängig voneinander für C₁-C₈-Alkyl-, C₁-C₈-Hydroxyalkyl- oder Benzyl-Gruppen stehen, und
- X für ein Anion wie Chlorid, Bromid, Methosulfat, Ethosulfat, Tosylat, Acetat, Lactat, Phosphat oder Nitrat steht.

Erfindungsgemäß bevorzugt sind solche Verbindungen der Formel (X), in denen
- R1, R2 und R3 für gleiche Reste stehen,
- X für Chlorid, Bromid oder Methosulfat steht,
- R bevorzugt für eine geradkettige Alkylengruppe -(CH2)n- steht, in der n = 1,2 oder 3 ist,
- Y für -N(R)-, insbesondere für -N(H)- oder für -N(CH₃) steht, und
- R4 eine Mischung verschiedener Kettenlängen von 8 bis 24 C-Atomen enthält, wie sie entstehen, wenn die Verbindungen der Formel (IV) aus natürlichen Rohstoffen, ausgewählt aus Talg-, Kokos-, Palmöl und/oder Soja, hergestellt werden.

Insbesondere bevorzugt sind Palmitamidopropyltrimoniumchlorid, Behenamidopropyltrimoniumchlorid, Palmitamidopropyltrimoniumbromid, Cocoamidopropyltrimoniumchlorid, Stearylamidopropyltrimonium-methosulfat, Talgamidopropyltrimoniumchlorid und Sojamidopropyltrimoniumchlorid.

In ganz besonders bevorzugten Ausführungsformen der Erfindung wird beispielsweise das unter der Handelbezeichnung "Varisoft® PATC" vertriebene Produkt der Formel (X) eingesetzt, das insbesondere in erfindungsgemäßen Haarreinigungsmitteln zu einer signifikanten Verbesserung des Griffs und der Kämmbarkeit der Haare beiträgt sowie die Geschmeidigkeit der Haare erhöht. Die Verbindungen der Formel (X) werden in den erfindungsgemäßen Haut- und/oder Haareinigungsmitteln - bezogen auf deren Gewicht - in einer Menge von 0,005 bis 10 Gew.-%, bevorzugt von 0,01 bis 7,5 Gew.-%, mehr bevorzugt von 0,05 bis 5 Gew.-% und insbesondere von 0,1 bis 3 Gew.-% eingesetzt.

In einer weiteren bevorzugten Ausführungsform der Erfindung sind die erfindungsgemäßen Haut- und/oder Haarreinigungsmitteln im Wesentlichen frei von weiteren Tensiden. Das bedeutet, dass sie nur die erfindungsgemäße Tensidmischung aus den Komponenten (a), (b) und (c) enthalten. Unter "im wesentlichen frei" ist erfindungsgemäß ein maximaler Gehalt von 4 Gew.-%, bevorzugt von 3 Gew.-%, mehr bevorzugt von 2,5 Gew.-% und insbesondere von 2 Gew.-% zu verstehen, wobei sich der maximale Gehalt weiterer Tenside auf das Gesamtgewicht der Haut- und/oder Haareinigungsmittel bezieht.

Diejenigen Tenside, die in untergeordneten Mengen in den erfindungsgemäßen Haut- und/oder Haareinigungsmitteln eingesetzt werden, sind meist Bestandteil eines Handelsprodukts.

Eine weitere fakultative aber bevorzugte erfindungsgemäße Komponente ist ein Konsistenzregulator, der in den erfindungsgemäßen Mitteln - bezogen auf deren Gewicht - in einer Menge von 0,01 bis 10 Gew.-%, bevorzugt von 0,05 bis 7,5 Gew.-%, mehr bevorzugt von 0,1 bis 5 Gew.-% und insbesondere von 0,5 bis 3 Gew.-% eingesetzt wird.

Bei diesen handelt es sich erfindungsgemäß um wasserlösliche und/oder teilweise quellbare natürliche oder synthetische Polymere, die in wässrigen Systemen Gele oder viskose Lösungen bilden. Geeignet sind beispielsweise organische, vollsynthetische Verbindungen, wie z. B. Polyacryl- und Polymethacryl-Verbindungen, Vinylpolymere, Polycarbonsäuren, Polyether, Polyimine, Polyamide, Polyurethane, anorganische Verbindungen, wie z. B. Polykieselsäuren, Tonmineralien wie Montmorillonite, Zeolithe, Kieselsäuren, organische, abgewandelte Naturstoffe, wie z. B. Carboxymethylcellulose und andere Celluloseether, Hydroxyethyl- und - propylcellulose und mikrokristalline Cellulose, sowie organische, natürliche Verbindungen, wie beispielsweise Agar-Agar, Carrageen, Tragant, Gummi arabicum, Alginate, Pektine, Laponite, Hectorite, Bentonite, Polyosen, Guar-Mehl, Johannisbrotbaumkernmehl, Stärke, Dextrine, Gelatine, Casein. In einer besonders bevorzugten Ausführungsform ist als Konsistenzregulator Xanthan (E 415) enthalten, auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen produziert. Xanthan wird aus einer Kette mit beta-1,4- gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Besonders geeignet ist beispielsweise Kelzan ASX-T (beziehbar von: CP Kelco).

Zur weiteren Verbesserung des Haut- und Haarzustandes enthalten erfindungsgemäße Haut- und/oder Haarbehandlungsmittel weiterhin bevorzugt mindestens ein weiteres Haut - und Haarkonditioniermittel, das aus der Gruppe der pflanzlichen Ölkomponenten, der Pflanzenextrakte und/oder den Proteinhydrolysaten ausgewählt ist.

Als Proteinhydrolysate im Sinne der Erfindung werden Proteinhydrolysate und/oder Aminosäuren und deren Derivate (H) verstanden. Proteinhydrolysate sind Produktgemische, die durch sauer, basisch oder enzymatisch katalysierten Abbau von Proteinen (Eiweißen) erhalten werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysaten. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Die Proteinhydrolysate oder deren Derivate sind in den erfindungsgemäßen Zubereitungen bevorzugt in Mengen von 0,01 bis 10 Gew.-%, bezogen auf das gesamte Mittel, enthalten. Mengen von 0,05 bis 7,5 und insbesondere 0,1 bis 5 Gew.-% sind besonders bevorzugt.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Babssuöl, Olivenöl, Weizenkeimöl, Kürbiskernöl, Malvenöl, Haselnussöl, Safloröl, Canolaöl, Sasanquaöl, Jojobaöl, Kakao Butter und Shea-Butter.

Die Pflanzenöle werden in den erfindungsgemäßen Mitteln in einer Menge von 0,01 bis 10 Gew.-%, bezogen auf das gesamte Mittel, eingesetzt. Mengen von 0,05 bis 7,5 und insbesondere 0,1 bis 5 Gew.-% sind besonders bevorzugt.

Erfindungsgemäß bevorzugt sind erfindungsgemäße Mittel, die Pflanzenextrakte aus grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Granatapfel, Mango, Aprikose, Limone, Weizen, Kiwi, Melone, Orange, Grapefruit, Holunder, Salbei, Rosmarin, Birke, Malve, Wiesenschaumkraut, Rooibos, Quendel, Schafgarbe, Thymian, Melisse, Hauhechel, Huflattich, Vanille, Eibisch, Meristem, Ginseng und Ingwerwurzel enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Die Pflanzenextrakte werden in den erfindungsgemäßen Mitteln - bezogen auf deren Gewichtin einer Menge von 0,01 bis 5 Gew.-%, vorzugsweise 0,02 bis 4 Gew.-% und insbesondere 0,05 bis 3 Gew.-% eingesetzt.

Die Pflegeffekte der erfindungsgemäßen Mittel lassen sich noch weiter verstärken, indem Pflege-Enhancer eingesetzt werden. Vorzugsweise werden diese aus bestimmten Gruppen an sich bekannter Pflegestoffe ausgewählt, da diese Pflege-Enhancer formulierungstechnisch und vom Pflegeffekt hervorragend mit der erfindungsgemäßen Tensidmischung harmonieren.

Erfindungsgemäß bevorzugte Haut- und/oder Haarreinigungsmittel sind dadurch gekennzeichnet, dass sie zusätzlich mindestens einen Pflege-Enhancer aus der Gruppe L-Carnitin und/oder seiner Salze; Panthenol und/oder Panthothensäure; der 2-Furanone und/oder deren Derivate, insbesondere Pantolacton; Taurin und/oder seiner Salze; Niacinamid; Ubichinon; Ectoin; Allantoin enthalten.

In erfindungsgemäßen Mitteln dieser Ausführungsform wird die Tensidmischung mit mindestens einem Pflege-Enhancer kombiniert, der ausgewählt ist aus L-Carnitin und/oder seinen Salzen, Panthenol und/oder Panthothensäure, 2-Furanonen und/oder deren Derivaten, insbesondere Pantolacton, Taurin und/oder seinen Salzen, Niacinamid, Ubichinonen, Ectoin, Allantoin, Extrakten von Echinacea. Diese Pflege-Enhancer werden nachstehend beschrieben L-Carnitin (IUPAC-Name(*R*)-(3-Carboxy-2-hydroxypropyl)- *N*,*N*,*N*-trimethylammoniumhydroxid), ist eine natürlich vorkommende, vitaminähnliche Substanz. Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH erhältlich.

Erfindungsgemäße bevorzugte Haut- und/oder Haarreinigungsmittel sind dadurch gekennzeichnet, dass sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Panthenol (IUPAC-Name: (+)-(R)-2,4-Dihydroxy-N-(3-hydroxypropyl)-3,3-dimethylbutyramid) wird im Körper zu Pantothensäure umgewandelt. Pantothensäure ist ein Vitamin aus der Gruppe der B-Vitamine (Vitamin B5).

Erfindungsgemäße bevorzugte Haut- und/oder Haarreinigungsmittel sind dadurch gekennzeichnet, dass sie - bezogen auf ihr Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol ((±)-2,4-Dihydroxy-*N*-(3-hydroxypropyl)-3,3-dimethyl-butyramid) enthalten.

Erfindungsgemäß bevorzugte Haut- und/oder Haarreinigungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% mindestens eines 2-Furanonderivats der Formel (Fur-I) und/oder der Formel (Fur-II) 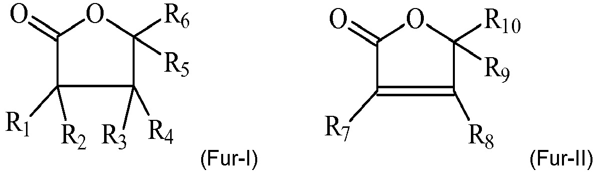 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen - C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.

In einer besonders bevorzugten Ausführungsform der erfindungsgemäßen Lehre wird eine Verbindung der Formel (Fur-I) eingesetzt. Dabei kann es bevorzugt sein, daß in einer Verbindung der Formel (Fur-I) die Reste R¹ und R² unabhängig voneinander stehen für:
- Wasserstoff, einen -OH-, einen Methyl-, Methoxy-, Aminomethyl-, Hydroxymethylrest,
- einen -C₂ - C₄ - gesättigten oder ein- oder zweifach ungesättigten, verzweigten oder linearen Mono-, Di- oder Trihydroxykohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen - C₂ - C₃₀ - gesättigten oder ein- oder mehrfach ungesättigten, verzweigten oder linearen Mono-, Di-, Tri- oder Polyhydroxykohlenwasserstoffrest.

In einer besonders bevorzugten Ausführungsform der erfindungsgemäßen Lehre wird als Verbindung entsprechend der Formel (Fur-I)
- (R)-(-)-4-Hydroxymethyl-Υ-butyrolacton und/oder
- D,L-4-Hydroxymethyl-γ-butyrolacton und/oder
- (S)-(+)-4-Hydroxymethyl-γ-butyrolacton und/oder
- R-(-)-2-Hydroxy-3,3-dimethyl-γ-butyrolacton und/oder
- D,L-2-Hydroxy-3,3-dimethyl-γ-butyrolacton und/oder
- S(+)-2-Hydroxy-3,3-dimethyl-γ-butyrolacton und/oder
- 4-Hydroxy-2,5-dimethyl-3(2H)-furanon und/oder
- Tetrahydro-5-oxo-2-furancarbonsäure und/oder
- Tetrahydro-5-oxo-2-furancarbonsäure, Na-Salz und/oder
- Tetrahydro-5-oxo-2-furancarbonsäure, K-Salz und/oder
- 2,5-Dihydro-5-methoxy-2-furanon und/oder
- Dihydro-3-hydroxy-4,4-dimethyl-2(3*H*)-furanon
eingesetzt. In einer ganz besonders bevorzugten Ausführungsform der erfindungsgemäßen Lehre wird als Verbindung entsprechend der Formel (Fur-I) Dihydro-3-hydroxy-4,4-dimethyl-2(3H)-furanon eingesetzt.

Ein weiterer, bevorzugter einsetzbarer Pflege-Enhancer, der aktivierende Eigenschaften besitzt, ist das Taurin. Erfindungsgemäß bevorzugte Haut- und/oder Haarreinigungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflege-Enhancerm in den erfindungsgemäßen Mitteln sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Haut- und/oder Haarreinigungsmittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-*N-*(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflege-Enhancer besitzen. Als weiteren Pflege-Enhancer können die erfindungsgemäßen Mittel daher 0,0001 bis 5 Gew.-% mindestens eines Biochinons der Formel (Ubi) 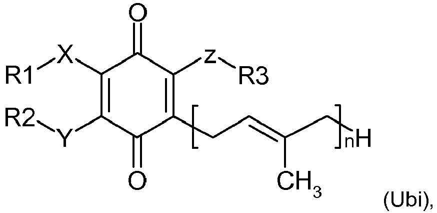 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, - CH₃, -CH₂CH₃, -(CH₂)₂CH₂, -CH(CH₃)₂, -(CH₂)₃CH₃, -CH(CH₃)CH₂CH₃, - CH₂CH(CH₃)₂, -C(CH₃)₃
- R⁴: steht für -CH₃, -CH₂CH₃, -(CH₂)₂CH₂, -CH(CH₃)₂, -(CH₂)₃CH₃, -CH(CH₃)CH₂CH₃, - CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte erfindungsgemäße Haut- und/oder Haarreinigungsmittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 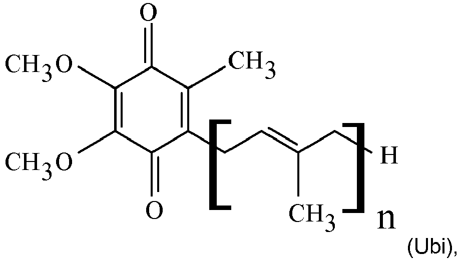 in der n für die Werte = 6, 7, 8, 9 oder 10, besonders bevorzugt für 10 (Coenzym Q10) steht.

Als weiteren Pflege-Enhacer können die erfindungsgemäßen Mittel Ectoin ((4S)-2-Methyl-1,4,5,6-Tetrahydropyrimidin-4-Carbonsäure) enthalten. Erfindungsgemäß bevorzugte Haarreinigungsmittel sind dadurch gekennzeichnet, daß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Ein weiterer Pflege-Enhancer ist Allantoin *(5-Ureidohydantoin, N*-(2,5-Dioxo-4-imidazolidinyl)-harnstoff), das in der Kosmetik in Hautcremes, Sonnenschutzmitteln, Rasierwässern, in Zahncremes und in Mitteln gegen übermäßige Schweißabsonderung (Hyperhidrose) und Hautirritationen eingesetzt wird. Es bewirkt die Beschleunigung des Zellaufbaus, der Zellbildung oder der Zellregeneration und beruhigt die Haut. Auch die Heilung schwer heilender Wunden wird unterstützt, jedoch besitzt Allantoin keine antiseptischen Eigenschaften.

Erfindungsgemäße besonders bevorzugte Haut- und/oder Haarreinigungsmittel enthalten - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% 5-Ureidohydantoin (Allantoin). Zur Verbesserung der Elastizität und Festigung der inneren Struktur der mit erfindungsgemäßen Mitteln behandelter Haare können die erfindungsgemäßen Mittel Purin und/oder Purinderivate als Pflege-Enhancer enthalten. Insbesondere die Kombination von Purin und/oder Purinderivaten mit Ubichinonen und/oder Plastochinonen als Pflege-Enhancer führt dazu, daß die mit entsprechenden Mitteln behandelten Haare unter anderem höhere Meßwerte bei der Differenzthermoanalyse und verbesserte Naß- und Trockenkämmbarkeiten zeigen.

Erfindungsgemäß bevorzugte kosmetische Mittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) der Formel (Pur-I) enthalten 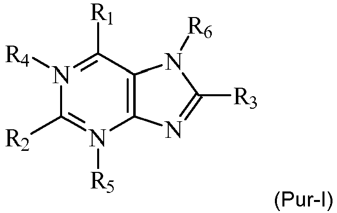 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflege-Enhancer können die erfindungsgemäßen Mittel auch Flavonoide enthalten. Erfindungsgemäß können Flavonoide aus allen sechs Gruppen eingesetzt werden, wobei bestimmte Vertreter aus den einzelnen Gruppen als Pflege-Enhancer wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Haut- und/oder Haarreinigungsmittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O*-rutinosid (Rutin), enthalten.

Bevorzugt ist auch der Einsatz von Bisabolol und/oder Bisabololoxiden als Pflege-Enhancer in den erfindungsgemäßen Mitteln. Hier sind erfindungsgemäße Haut- und/oder Haarreinigungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflege-Enhancer. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Haut- und/oder Haarreinigungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% *N*-Methyl-guanidino-essigsäure (Creatin). Die erfindungsgemäßen Mittel können (sofern sie als Haarreinigungsmittel konfektioniert werden) zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben:
Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann.

Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich. Zur Bekämpfung der hormonellen Einflüße auf die Haarfollikel kann zur äußeren Anwendung Spironolactone in Form von Haarwasser und in Kombination mit Minoxidil angewandt werden. Spironolactone wirkt als Androgen-Rezeptor-Blocker, dh. die Bindung von DHT an die Haarfollikel wird verhindert.

Zusammenfassend sind erfindungsgemäße Haarbehandlungsmittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Durch zusätzliche Antischuppenwirkstoffe wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, daß die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.

Zusammenfassend sind erfindungsgemäße Haarreinigungsmittel bevorzugt, die Antischuppenmittel in einer Menge von 0,05 bis 5 Gew.-%, bevorzugt von 0,1 bis 3,0 Gew.-% und insbesondere von 0,3 bis 2,0 Gew.-% enthalten (bezogen auf das gesamte Mittel). Bevorzugte Antischuppenmittel sind ausgewählt aus Piroctone Olamine, Climbazol, Zink Pyrithion, Ketoconazole, Salicylsäure, Schwefel, Selensulfide, Teerpräparaten, Undecensäurederivaten, Klettenwurzel-, Pappel-, Brennessel-, Walnussschalen-, Birken-, Weidenrinden-, Rosmarin- und/oder Arnikaextrakten.

Erfindungsgemäß bevorzugt sind Salicylsäure, Climbazol, Zink Pyrithion und Piroctone Olamine. Zusätzlich zu den Pflege-Enhancern können di erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3',4'-Dihydroxy-L-phenylalanin (L-Dopa), 5 '-Hydroxy-L-tryptophan, L-Homocystein, *S*-Methyl-L-methionin, *S*-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haut- und/oder Haarreinigungsmittel dadurch gekennzeichnet, daß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haut- und/oder Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus
- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

Eine besonders bevorzugte Gruppe von Inhaltsstoffen stellen die Silikone dar. Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Mit Vorzug sind die Silikone wasserlöslich. Erfindungsgemäß bevorzugte Mittel sind dadurch gekennzeichnet, daß sie mindestens ein wasserlösliches Silikon enthalten.

Aus ästhetischen Gründen werden "klare" Produkte von Verbrauchern oft bevorzugt. Erfindungsgemäß bevorzugte Haarbehandlungsmittel sind daher dadurch gekennzeichnet, daß sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Bevorzugt sind solche UV-Filter, deren molarer Extinktionskoeffizient am Absorptionsmaximum oberhalb von 15 000, insbesondere oberhalb von 20000, liegt.

Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyl-dimethylaminobenzamidopropyl-dimethylammoniumtosylat (Escalol® HP 610).

Die UV-Filter (I) sind in den erfindungsgemäßen Mitteln üblicherweise in Mengen 0,1-5 Gew.-%, bezogen auf das gesamte Mittel, enthalten. Mengen von 0,4-2,5 Gew.-% sind bevorzugt.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß verwendeten Carbonsäuren können selbstverständlich entlang der Kohlenstoffkette oder des Ringgerüstes substituiert sein. Zu den Substituenten der erfindungsgemäß verwendeten Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in □ - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Gemische aus Alkyl-(oligo)-glucosiden und Fettalkoholen zum Beispiel das im Handel erhältliche Produkt Montanov®68,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

In einer bevorzugten Ausführungsform werden den erfindungsgemäßen Mitteln weiterhin Polymere zugesetzt, wobei der Einsatz kationischer Polymere, die eine haut- und haarkonditionierende Wirkung aufweisen, bevorzugt ist.

Unter erfindungsgemäß geeigneten kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (VI), 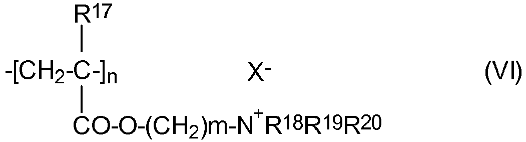 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2,
- X⁻ steht für Halogenid-, Sulfat-, Phosphat-, Methosulfat- sowie organische Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte, Poly(methacryloyloxyethyl-trimethylammoniumchlorid) (INCI-Bezeichnung Polyquaternium-37). Ein entsprechendes, geeignetes Handelsprodukt ist unter den Bezeichnungen Salcare® SC 95 (INCI-Bezeichnung: Mineral Oil) und Tridecyl-polyoxypropylen-polyoxyethylen-ether (INCI-Bezeichnung: PPG-1-Trideceth-6)) und Salcare® SC 96 (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Ein erfindungsgemäß bevorzugtes Copolymer ist das vernetzte Acrylamid-Methacryloyloxyethyltrimethylammoniumchlorid-Copolymer, wie beispielsweise das im Handel unter der Bezeichnung Salcare® SC 92 erhältliche Copolymer.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure wie beispielsweise die Handelsprodukte Merquat®100 (Poly(dimethyldiallylammoniumchlorid)) und Merquat®550,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

In einer besonders bevorzugten Ausführungsform der Erfindung ist als kationisches Polymer mindestens ein Polymer aus der Gruppe der kationischen Guar-Derivate und/oder Polyquaternium-7 (Merquat 550), Polyquaternium-6, Polyquaternium-10 und/oder Polyquaternium-67 (SoftCat®-Polymere) in den erfindungsgemäßen Mitteln enthalten.

Das oder die kationische(n) Polymer(e) ist (sind) in den erfindungsgemäßen Zusammensetzungen - bezogen auf die gesamte Zusammensetzung - in Mengen von 0,1 bis 5 Gew.-% enthalten. Mengen von 0,2 bis 3, insbesondere von 0,5 bis 2 Gew.-%, sind besonders bevorzugt.

Ein zweiter Gegenstand der vorliegenden Erfindung ist die Verwendung einer besonders milden Tensidkombination aus
a. mindestens einem Acylaminosäuresalz der zuvor genannten Formel (VI)
b. mindestens einem Alkylpolyglucosid und
c. mindestens einem zwitterionischen und/oder amphoteren Tensid,
in einem Verhältnis der Komponenten (a) : (b): (c) von (0,1-1) : (1-3): (0,1-5)
in kosmetischen Haut- und/oder Haarbehandlungsmitteln, die sulfatfrei sind, zur Verbesserung der Hautverträglichkeit.

Ein dritter Gegenstand der Erfindung ist die Verwendung einer besonders milden Tensidkombination aus
a. mindestens einem Acylaminosäuresalz der zuvor genannten Formel (VI),
b. mindestens einem Alkylpolyglucosid und
c. mindestens einem zwitterionischen und/oder amphoteren Tensid,
in einem Verhältnis der Komponenten (a) : (b) : (c) von (0,1-1) : (1-3): (0,1-5) zur Herstellung eines sulfatfreien kosmetischen Haut- und/oder Haarbehandlungsmittels, das einen besonders feinporigen und cremigen Schaum bildet.

Ein vierter Gegenstand der Erfindung ist die Verwendung einer besonders milden Tensidkombination aus
a. mindestens einem Acylaminosäuresalz der zuvor genannten Formel (VI)
b. mindestens einem Alkylpolyglucosid,
c. mindestens einem zwitterionischen und/oder amphoteren Tensid und
d. mindestens einer Verbindung der Formel (X), 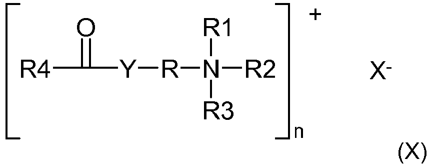 in der
   - R4 für eine lange aliphatische Kohlenstoffgruppe mit 8 bis 24 C-Atomen steht,
   - Y für ein Heteroatom, ausgewählt aus O, N oder S steht, wobei im Falle von Y=N das Stickstoffatom noch einen Substituenten -H oder -C₁-C₄-Alkyl trägt,
   - R für eine geradekettige oder verzweigte Alkylengruppe mit 1 bis 10 C-Atomen steht,
   - R1, R2 und R3 unabhängig voneinander für C₁-C₈-Alkyl-, C₁-C₈-Hydroxyalkyl- oder Benzyl-Gruppen stehen, und
   - X für ein Anion wie Chlorid, Bromid, Methosulfat, Ethosulfat, Tosylat, Acetat, Lactat, Phosphat oder Nitrat steht,
zur Verbesserung des Griffs, der Kämmbarkeit und der Geschmeidigkeit keratinischer Fasern. Beschrieben wird auch Verfahren zur Reinigung und Pflege menschlicher Haut und menschlicher Haare, bei dem ein erfindungsgemäßes Mittel auf die nassen Haare aufgetragen und nach einer Einwirkungszeit von 10 Sekunden bis 5 Minuten wieder ausgespült wird.

Die folgenden Beispiele erläutern die Erfindung, ohne sie jedoch darauf einzuschränken: Alle Angaben sind - sofern nicht anders angegeben - in Gewichtsprozent.

### Beispiele:

### 1) Shampoo

| | **Menge** |
|---|---|
| **Coco-Glucoside** | 5,2 |
| **Sodium Cocoamphoacetate** | 4,7 |
| **Disodium Cocoyl Glutamate** | 4,0 |
| **Citric Acid** | 1,6 |
| **Sodium Chloride** | 1,0 |
| **Xanthan Gum** | 1,0 |
| **Palmitamidopropyltrimonium Chloride** | 0,6 |
| **Sodium Benzoate** | 0,5 |
| **Propylene Glycol** | 0,4 |
| **Salicylic Acid** | 0,2 |
| **Parfum** | 0,2 |
| **Aqua** | ad 100 |

### 2) Haarkonditioniershampoo

| | **Menge** |
|---|---|
| **Coco-Glucoside** | 5,2 |
| **Cocamidopropyl Betaine** | 7,6 |
| **Disodium Cocoyl Glutamate** | 4,0 |
| **Citric Acid** | 1,6 |
| **Sodium Chloride** | 1,0 |
| **Xanthan Gum** | 1,0 |
| **Palmitamidopropyltrimonium Chloride** | 0,6 |
| **Laurdimonium Hydroxypropyl Hydrolyzed Wheat Protein** | 0,5 |
| **Sodium Benzoate** | 0,5 |
| **Propylene Glycol** | 0,4 |
| **Salicylic Acid** | 0,2 |
| **Parfum** | 0,2 |
| **Preservatives** | 0,05 |
| **Aqua** | ad 100 |

## Patentansprüche

1. Sulfat-freies Haut- und/oder Haarreinigungsmittel, enthaltend in einem kosmetisch akzeptablen Träger eine milde Tensidmischung aus
(a) mindestens einem Acylaminosäuresalz, das ausgewählt ist aus Verbindungen der Formel (VI), 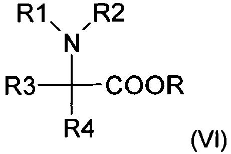 in der
- der Rest R für ein Wasserstoffatom, ein Alkali- oder Erdalkalimetallion, ein Ammonium-, Alkylammonium- oder Alkanolammoniumion steht,
- einer der Reste R1 oder R2 für Wasserstoff, eine Phenyl- oder eine -CH₂-COOR-Gruppe, und der andere Rest R1 oder R2 für eine COR'-Gruppe steht, in der R' für einen geradkettigen oder verzweigten, gesättigten oder ungesättigten Alkylrest mit 8 bis 30 C-Atomen steht,
- die Reste R3 und R4 unabhängig voneinander für Wasserstoff- oder lineare oder verzweigte Gruppen -CH(R5)-(CH₂)ₙ-COOR stehen, in denen R5 Wasserstoff oder eine C₁-C₄-Alkylgruppe ist und n für eine ganze Zahl von 0 bis 10 steht.
(b) mindestens einem Alkylpolyglucosid und
(c) mindestens einem zwitterionischen und/oder amphoteren Tensid,
**dadurch gekennzeichnet, dass** das Verhältnis der Komponenten (a) : (b) : (c) im Bereich von (0,1-1) : (1-3): (0,1-5) beträgt.

2. Haut- und/oder Haarreinigungsmittel nach Anspruch 1, **dadurch gekennzeichnet, dass** es frei von Verbindungen der folgenden Formeln ist,
**H-(O-CH₂CH₂)ₙ-OH** **(I),**
in der n für ganze Zahlen zwischen 1 und 100.000 steht,
**H-(O-CH(CH₃)CH₂)ₙ-OH** **(II),**
in der n für ganze Zahlen zwischen 1 und 100.000 steht,
**R-(O-CH₂CH₂)ₙ-OH** **(III),**
in der R für jedweden Alkyl- oder Alkenylrest und n für ganze Zahlen zwischen 1 und 10.000 steht,
**R-(O-CH₂CH₂)ₙ-OSO₃H** **(IV),**
in der R für jedweden Alkyl- oder Alkenylrest und n für ganze Zahlen zwischen 1 und 10.000 steht, und
**-(O-CH₂CH₂)ₙ-** **(V),**
in der n für ganze Zahlen zwischen 2 und 100.000 steht.

3. Haut- und/oder Haarreinigungsmittel nach einem der Ansprüche 1 oder 2, **dadurch gekennzeichnet, dass** es die Komponenten (a), (b) und (c) - jeweils bezogen auf das Gesamtgewicht des Mittels - in den folgenden Mengen aufweist:
(a) 0,001 bis 15 Gew.-%, bevorzugt 0,005 bis 10 Gew.-%, mehr bevorzugt 0,01 bis 7,5 Gew.-% und insbesondere 0,05 bis 5 Gew.-%,
(a) 0,05 bis 15 Gew.-%, bevorzugt 0,1 bis 12,5 Gew.-%, mehr bevorzugt 0,5 bis 10 Gew.-% und insbesondere 1 bis 7,5 Gew.-%,
(b) 0,05 bis 20 Gew.-%, bevorzugt 0,1 bis 15 Gew.-%, mehr bevorzugt 0,5 bis 12,5 Gew.-% und insbesondere 1 bis 10 Gew.-%.

4. Haut- und/oder Haarreinigungsmittel nach einem der Ansprüche 1 bis 4 3, **dadurch gekennzeichnet, dass** es amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthält.

5. Haut- und/oder Haarreinigungsmittel nach einem der Ansprüche 1 bis 4, **dadurch gekennzeichnet, dass** es zusätzlich eine Verbindung der Formel (X) enthält, 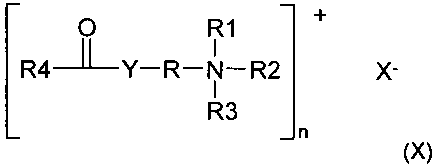 in der
- R4 für eine lange aliphatische Kohlenstoffgruppe mit 8 bis 24 C-Atomen steht,
- Y für ein Heteroatom, ausgewählt aus O, N oder S steht, wobei im Falle von Y=N das Stickstoffatom noch einen Substituenten -H oder -C₁-C₄-Alkyl trägt,
- R für eine geradekettige oder verzweigte Alkylengruppe mit 1 bis 10 C-Atomen steht,
- R1, R2 und R3 unabhängig voneinander für C₁-C₈-Alkyl-, C₁-C₈-Hydroxyalkyl- oder Benzyl-Gruppen stehen, und
- X für ein Anion wie Chlorid, Bromid, Methosulfat, Ethosulfat, Tosylat, Acetat, Lactat, Phosphat oder Nitrat steht.

6. Haut- und/oder Haarreinigungsmittel nach Anspruch 6 5, **dadurch gekennzeichnet, dass** es - bezogen auf sein Gesamtgewicht - 0,005 bis 10 Gew.-%, bevorzugt 0,01 bis 7,5 Gew.-%, mehr bevorzugt 0,05 bis 5 Gew.-% und insbesondere 0,1 bis 3 Gew.-% Verbindungen der Formel (X) enthält, in denen bevorzugt
• R1, R2 und R3 für gleiche Reste stehen,
• X für Chlorid, Bromid oder Methosulfat steht,
• R bevorzugt für eine geradkettige Alkylengruppe -(CH2)n- steht, in der n = 1,2,3 ist,
• Y für -N(R)-, insbesondere für -N(H)- oder für -N(CH₃) steht, und
• R4 eine Mischung verschiedener Kettenlängen von 8 bis 24 C-Atomen enthält, wie sie entstehen, wenn die Verbindungen der Formel (X) aus natürlichen Rohstoffen, ausgewählt aus Talg-, Kokos-, Palmöl und/oder Soja, hergestellt werden.

7. Haut- und/oder Haarreinigungsmittel nach einem der Ansprüche 1 bis 6, **dadurch gekennzeichnet, dass** es als Reinigungs-Basis im wesentlichen nur die Tensidmischung aus den Komponenten (a), (b) und (c) enthält.

8. Haut- und/oder Haarreinigungsmittel nach einem der Ansprüche 1 bis 7, **dadurch gekennzeichnet, dass** es zusätzlich mindestens einen Konsistenzregulator enthält, der bevorzugt aus der Gruppe der natürlichen Konsistenzregulatoren ausgewählt ist.

9. Haut- und/oder Haarreinigungsmittel nach einem der Ansprüche 1 bis 8, **dadurch gekennzeichnet, dass** es weiterhin mindestens eine Komponente aus der Gruppe der pflanzlichen Ölkomponenten, der Pflanzenextrakte und/oder der Proteinhydrolysate enthält.

10. Verwendung einer besonders milden Tensidkombination aus
a. mindestens einem Acylaminosäuresalz der zuvor genannten Formel (VI),
b. mindestens einem Alkylpolyglucosid und
c. mindestens einem zwitterionischen und/oder amphoteren Tensid,
in einem Verhältnis der Komponenten (a) : (b) : (c) von (0,1-1) : (1-3) : (0,1-5) in kosmetischen Haut- und/oder Haarbehandlungsmitteln, die sulfatfrei sind, zur Verbesserung der Hautverträglichkeit.

11. Verwendung einer besonders milden Tensidkombination aus
a. mindestens einem Acylaminosäuresalz der zuvor genannten Formel (VI),
b. mindestens einem Alkylpolyglucosid und
c. mindestens einem zwitterionischen und/oder amphoteren Tensid,
in einem Verhältnis der Komponenten (a) : (b) : (c) von (0,1-1) : (1-3) : (0,1-5)
zur Herstellung eines sulfatfreien kosmetischen Haut- und/oder Haarbehandlungsmittels, das einen besonders feinporigen und cremigen Schaum bildet.

12. Verwendung eines Sulfat-freien Haut- und/oder Haarreinigungsmittels, basierend auf einer besonders milden Tensidkombination aus
a. mindestens einem Acylaminosäuresalz der zuvor genannten Formel (VI),
b. mindestens einem Alkylpolyglucosid,
c. mindestens einem zwitterionischen und/oder amphoteren Tensid und
d. mindestens einer Verbindung der Formel (VII), 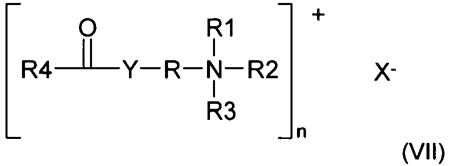 in der
• R4 für eine lange aliphatische Kohlenstoffgruppe mit 8 bis 24 C-Atomen steht,
• Y für ein Heteroatom, ausgewählt aus O, N oder S steht, wobei im Falle von Y=N das Stickstoffatom noch einen Substituenten -H oder -C₁-C₄-Alkyl trägt,
• R für eine geradekettige oder verzweigte Alkylengruppe mit 1 bis 10 C-Atomen steht,
• R1, R2 und R3 unabhängig voneinander für C₁-C₈-Alkyl-, C₁-C₈-Hydroxyalkyl- oder Benzyl-Gruppen stehen, und
• X für ein Anion wie Chlorid, Bromid, Methosulfat, Ethosulfat, Tosylat, Acetat, Lactat, Phosphat oder Nitrat steht,
zur Verbesserung des Griffs, der Kämmbarkeit und der Geschmeidigkeit keratinischer Fasern.

## Claims

1. A sulfate-free skin and/or hair cleansing agent containing, in a cosmetically acceptable carrier, a mild surfactant mixture, consisting of
(a) at least one acylamino acid salt which is selected from compounds of formula (VI), 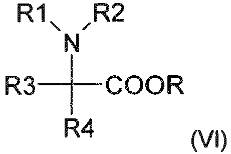 in which
- the functional group R represents a hydrogen atom, an alkali metal ion or alkaline-earth metal ion, an ammonium ion, alkyl ammonium ion or alkanol ammonium ion,
- one of the functional groups R1 or R2 represents hydrogen, a phenyl group or -CH₂-COOR group, and the other functional group R1 or R2 represents a COR' group, in which R' represents a straight-chain or branched, saturated or unsaturated alkyl functional group having 8 to 30 C atoms,
- the functional groups R3 and R4 represent, independently of one another, hydrogen or linear or branched groups of -CH(R5)-(CH₂)ₙ-COOR, in which R5 is hydrogen or a C₁-C₄ alkyl group and n represents an integer of from 0 to 10.
(b) at least one alkyl polyglucoside and
(c) at least one zwitterionic and/or amphoteric surfactant,
**characterized in that** the ratio of the components (a) : (b) : (c) is in the range of (0.1-1) : (1-3) : (0.1-5).

2. The skin and/or hair cleansing agent according to claim 1, **characterized in that** it is free of compounds of the following formulas:
H-(O-CH₂CH₂)ₙ-OH (I),
in which n represents integers between 1 and 100,000,
H-(O-CH(CH₃)CH₂)ₙ-OH (II),
in which n represents integers between 1 and 100,000,
R-(O-CH₂CH₂)ₙ-OH (III),
in which R represents any alkyl or alkenyl functional group and n represents integers between 1 and 10,000,
R-(O-CH₂CH₂)ₙ- OSO₃H (IV),
in which R represents any alkyl or alkenyl functional group and n represents integers between 1 and 10,000, and
-(O-CH₂CH₂)ₙ- (V),
in which n represents integers between 2 and 100,000.

3. The skin and/or hair cleansing agent according to either claim 1 or claim 2, **characterized in that** it comprises the components (a), (b), and (c) in the following amounts, based in each case on the total weight of the agent:
(a) 0.001 to 15 wt.%, preferably 0.005 to 10 wt.%, more preferably 0.01 to 7.5 wt.% and in particular 0.05 to 5 wt.%,
(b) 0.05 to 15 wt.%, preferably 0.1 to 12.5 wt.%, more preferably 0.5 to 10 wt.% and in particular 1 to 7.5 wt.%,
(c) 0.05 to 20 wt.%, preferably 0.1 to 15 wt.%, more preferably 0.5 to 12.5 wt.% and in particular 1 to 10 wt.%.

4. The skin and/or hair cleansing agent according to one of claims 1 to 3, **characterized in that** it contains amphoteric surfactant(s) from the groups of the
- N-alkylglycines,
- N-alkylpropionic acids,
- N-alkylaminobutyric acids,
- N-alkyliminodipropionic acids,
- N-hydroxyethyl-N-alkylamidopropylglycines,
- N-alkyl taurines,
- N-alkyl sarcosines,
- 2-alkylaminopropionic acids having in each case approximately 8 to 24 C atoms in the alkyl group,
- alkylaminoacetic acids having in each case approximately 8 to 24 C atoms in the alkyl group,
- N-cocoalkyl aminopropionate,
- cocoacylaminoethyl aminopropionate,
- C₁₂-C₁₈ - acyl sarcosine,
- N-alkyl-N,N-dimethylammonium glycinates, for example cocoalkyl dimethylammonium glycinate,
- N-acylaminopropyl-N,N-dimethylammonium glycinates, for example cocoacylaminopropyl dimethylammonium glycinate,
- 2-alkyl-3-carboxymethyl-3-hydroxyethyl-imidazolines each having 8 to 18 C atoms in the alkyl or acyl group,
- cocoacylaminoethyl hydroxyethyl carboxymethyl glycinate,
- the compounds known by the INCI designation Cocamidopropyl Betaine, - the compounds known by the INCI designation Disodium Cocoamphodiacetate.

5. The skin and/or hair cleansing agent according to one of claims 1 to 4, **characterized in that** it additionally contains a compound of formula (X), 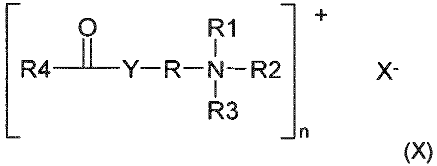 in which
- R4 represents a long aliphatic carbon group having 8 to 24 C atoms,
- Y represents a heteroatom selected from O, N or S, the nitrogen atom carrying another substituent -H or -C₁-C₄alkyl if Y=N,
- R represents a straight-chain or branched alkylene group having 1 to 10 C atoms,
- R1, R2 and R3 represent, independently of one another, C₁-C₈ alkyl groups, C₁-C₈ hydroxyalkyl groups or benzyl groups, and
- X represents an anion such as chloride, bromide, methosulfate, ethosulfate, tosylate, acetate, lactate, phosphate or nitrate.

6. The skin and/or hair cleansing agent according to claim 5, **characterized in that** it contains, based on the total weight thereof, 0.005 to 10 wt.%, preferably 0.01 to 7.5 wt.%, more preferably 0.05 to 5 wt.% and in particular 0.1 to 3 wt.% of compounds of formula (X), in which, preferably,
• R1, R2 and R3 represent the same functional groups,
• X represents chloride, bromide or methosulfate,
• R preferably represents a straight-chain alkylene group -(CH₂)ₙ-, in which n = 1, 2 or 3,
• Y represents -N(R)-, in particular -N(H)- or -N(CH₃), and
• R4 contains a mixture of different chain lengths of 8 to 24 C atoms, as they arise, if the compounds of formula (X) are prepared from natural raw materials selected from tallow oil, coconut oil, palm oil and/or soy.

7. The skin and/or hair cleansing agent according to one of claims 1 to 6, **characterized in that** it substantially only contains the surfactant mixture of the components (a), (b) and (c) as the cleansing base.

8. The skin and/or hair cleansing agent according to one of claims 1 to 7, **characterized in that** it additionally contains at least one consistency regulator which is preferably selected from the group of the natural consistency regulators.

9. The skin and/or hair cleansing agent according to one of claims 1 to 8, **characterized in that** it further contains at least one component from the group of the vegetal oil components, plant extracts and/or protein hydrolyzates.

10. The use of a particularly mild surfactant combination of
a. at least one acylamino acid salt of the aforementioned formula (VI),
b. at least one alkyl polyglucoside and
c. at least one zwitterionic and/or amphoteric surfactant,
in a ratio of the components (a) : (b) : (c) of (0.1-1) : (1-3): (0.1-5) in cosmetic skin and/or hair treatment agents, which are sulfate-free, for improving the skin compatibility.

11. The use of a particularly mild surfactant combination of
a. at least one acylamino acid salt of the aforementioned formula (VI),
b. at least one alkyl polyglucoside and
c. at least one zwitterionic and/or amphoteric surfactant,
in a ratio of the components (a) : (b) : (c) of (0.1-1) : (1-3) : (0.1-5) for preparing a sulfate-free cosmetic skin and/or hair treatment agent
which forms a particularly fine-pored and creamy mousse.

12. The use of a sulfate-free skin and/or hair cleansing agent, based on a particularly mild surfactant combination of
a. at least one acylamino acid salt of the aforementioned formula (VI),
b. at least one alkyl polyglucoside,
c. at least one zwitterionic and/or amphoteric surfactant and
d. at least one compound of formula (VII) 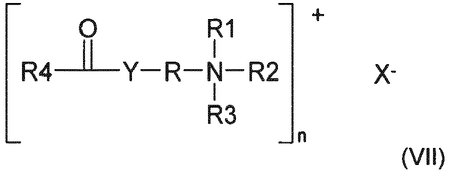 in which
• R4 represents a long aliphatic carbon group having 8 to 24 C atoms,
• Y represents a heteroatom selected from O, N or S, the nitrogen atom carrying another substituent -H or -C₁-C₄ alkyl if Y=N,
• R represents a straight-chain or branched alkylene group having 1 to 10 C atoms,
• R1, R2 and R3 represent, independently of one another, C₁-C₈ alkyl groups, C₁-C₈ hydroxyalkyl groups or benzyl groups, and
• X represents an anion such as chloride, bromide, methosulfate, ethosulfate, tosylate, acetate, lactate, phosphate or nitrate,
for improving the feel, the combability and the smoothness of keratin fibers.

## Revendications

1. Agent nettoyant capillaire et/ou cutané sans sulfate contenant, dans un support cosmétiquement acceptable, un mélange de tensioactifs doux constitué
(a) d'au moins un sel de d'acide acylaminé choisi parmi les composés de la formule (VI), 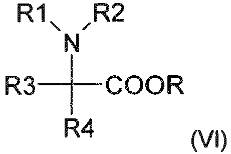 dans laquelle
- le radical R est un atome d'hydrogène, un ion de métal alcalin ou alcalino-terreux, un ion ammonium, alkylammonium ou alcanolammonium,
- l'un des radicaux R1 ou R2 est un atome d'hydrogène, un groupe phényle ou un groupe -CH₂-COOR, et l'autre radical R1 ou R2 est un groupe COR' dans lequel R' est radical alkyle saturé ou insaturé, linéaire ou ramifié, ayant de 8 à 30 atomes de carbone,
- les radicaux R3 et R4 sont indépendamment l'hydrogène ou des groupements linéaires ou ramifiés -CH(R5)-(CH₂)ₙ-COOR, dans lesquels R5 est l'hydrogène ou un groupe alkyle en C₁ à C₄ et n est un nombre entier de 0 à 10,
(b) d'au moins un alkylpolyglucoside et
(c) d'au moins un tensioactif zwittérionique et/ou amphotère,
**caractérisé en ce que** le rapport des composants (a):(b):(c) est dans la gamme de (0,1 à 1):(1 à 3):(0,1 à 5).

2. Agent nettoyant capillaire et/ou cutané selon la revendication 1, **caractérisé en ce qu'**il est exempt de composés des formules suivantes
H-(O-CH₂CH₂)ₙ-OH (I),
où n représente des nombres entiers compris entre 1 et 100000,
H-(O-CH(CH₃)CH₂)ₙ-OH (II),
où n représente des nombres entiers compris entre 1 et 100000,
R-(O-CH₂CH₂)ₙ-OH (III),
où R représente tout radical alkyle ou alcényle et n représente des nombres entiers compris entre 1 et 10000,
R-(O-CH₂CH₂)ₙ-OSO₃H (IV),
où R représente tout radical alkyle ou alcényle et n représente des nombres entiers compris entre 1 et 10000, et
-(O-CH₂CH₂)ₙ- (V),
où n représente des entiers compris entre 2 et 100000.

3. Agent nettoyant capillaire et/ou cutané selon la revendication 1 ou la revendication 2, **caractérisé en ce qu'**il contient les composants (a), (b) et (c), à chaque fois par rapport au poids total de l'agent, dans les proportions suivantes :
(a) de 0,001 à 15% en poids, de préférence de 0,005 à 10% en poids, plus préférablement de 0,01 à 7,5% en poids et en particulier de 0,05 à 5% en poids,
(b) de 0,05 à 15% en poids, de préférence de 0,1 à 12,5% en poids, plus préférablement de 0,5 à 10% en poids et en particulier de 1 à 7,5% en poids,
(c) de 0,05 à 20% en poids, de préférence de 0,1 à 15% en poids, plus préférablement de 0,5 à 12,5% en poids et en particulier de 1 à 10% en poids.

4. Agent nettoyant capillaire et/ou cutané selon l'une des revendications 1 à 3, **caractérisé en ce qu'**il contient un ou plusieurs tensioactifs amphotères des groupes comportant
- les N-alkylglycines,
- les acides N-alkylpropioniques,
- les acides N-alkylaminobutyriques,
- les acides N-alkyliminodipropioniques,
- les N-hydroxyéthyl-N-alkylamidopropylglycines,
- les N-alkyltaurines
- les N-alkylsarcosines,
- les acides 2-alkylaminopropioniques ayant chacun environ de 8 à 24 atomes de carbone dans le groupe alkyle,
- les acides alkylaminoacétiques ayant chacun environ 8 à 24 atomes de carbone dans le groupe alkyle,
- le N-cocoalkylaminopropionate,
- le cocoacylaminoéthylaminopropionate
- l'acylsarcosine en C12 à C18,
- les glycinates de N-alkyl-N,N-diméthylammonium, par exemple le glycinate de cocoalkyl-diméthyl-ammonium,
- les glycinates de N-acylaminopropyl-N,N-diméthylammonium, par exemple le glycinate de cocosacylaminopropyl-diméthylammonium,
- les 2-alkyl-3-carboxyméthyl-3-hydroxyéthyl-imidazolines contenant de 8 à 18 atomes de carbone dans le groupe alkyle ou acyle,
- le cocosacylaminoéthylhydroxyéthylcarboxyméthylglycinate
- les composés connus sous la désignation INCl Cocamidopropyl Betain
- les composés connus sous la désignation INCl Disodium Cocoamphodiacetate.

5. Agent nettoyant capillaire et/ou cutané selon l'une des revendications 1 à 5, **caractérisé en ce qu'**il contient en outre un composé de la formule (X) 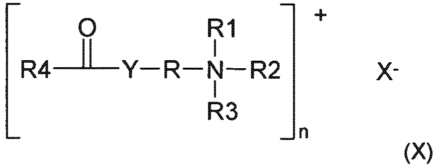 dans laquelle
- R4 est un long groupe carboné aliphatique ayant de 8 à 24 atomes de carbone,
- Y est un hétéroatome choisi parmi O, N ou S, dans le cas où Y=N l'atome d'azote porte encore un substituant -H ou -alkyle en C₁ à C₄,
- R est un groupe alkylène linéaire ou ramifié ayant de 1 à 10 atomes de carbone,
- R1, R2 et R3 sont indépendamment un groupe alkyle en C₁ à C₈, hydroxyalkyle en C₁ à C₈ ou benzyle, et
- X est un anion tel qu'un chlorure, un bromure, un méthosulfate, un éthosulfate, un tosylate, un acétate, un lactate, un phosphate ou un nitrate.

6. Agent nettoyant capillaire et/ou cutané selon la revendication 5, **caractérisé en ce qu'**il contient, par rapport à son poids total, de 0,005 à 10% en poids, de préférence de 0,01 à 7,5% en poids, plus préférablement de 0,05 à 5% en poids et en particulier 0,1 à 3% en poids de composés de la formule (X) où de préférence
• R1, R2 et R3 sont les mêmes radicaux,
• X est un chlorure, un bromure ou un méthosulfate,
• R est de préférence un groupe alkylène linéaire -(CH2)n-, où n est 1, 2, 3,
• Y est -N(R)-, en particulier -N(H)- ou -N(CH₃), et
• R4 contient un mélange de différentes longueurs de chaîne de 8 à 24 atomes de carbone qui se forment lorsque les composés de la formule (X) sont préparés à partir de matières premières naturelles choisies parmi le suif, l'huile de coco, l'huile de palme et/ou le soja.

7. Agent nettoyant capillaire et/ou cutané selon l'une des revendications 1 à 6, **caractérisé en ce qu'**il ne contient sensiblement que le mélange de tensioactifs des composants (a), (b) et (c) comme base nettoyante.

8. Agent nettoyant capillaire et/ou cutané selon l'une des revendications 1 à 7, **caractérisé en ce qu'**il contient en outre au moins un régulateur de consistance choisi de préférence dans le groupe des régulateurs de consistance naturels.

9. Agent nettoyant capillaire et/ou cutané selon l'une des revendications 1 à 8, **caractérisé en ce qu'**il contient en outre au moins un composant du groupe comportant des composants d'huiles végétales, des extraits de plantes et/ou des hydrolysats de protéines.

10. Utilisation d'une combinaison de tensioactifs particulièrement doux constituée
a. d'au moins un sel de d'acide acylaminé de la formule susmentionnée (VI),
b. d'au moins un alkylpolyglucoside et
c. d'au moins un tensioactif zwittérionique et/ou amphotère,
dans un rapport des composants (a):(b):(c) de (0,1 à 1):(1 à 3):(0,1 à 5) dans des agents de traitement cosmétique capillaire et/ou cutané, qui sont exempts de sulfate, pour améliorer la compatibilité cutanée.

11. Utilisation d'une combinaison de tensioactifs particulièrement doux constituée
a. d'au moins un sel de d'acide acylaminé de la formule susmentionnée (VI),
b. d'au moins un alkylpolyglucoside et
c. d'au moins un tensioactif zwittérionique et/ou amphotère,
dans un rapport des composants (a):(b):(c) de (0,1 à 1):(1 à 3):(0,1 à 5) pour la fabrication d'un agent de traitement cosmétique capillaire et/ou cutané, exempt de sulfate, qui forme une mousse notamment crémeuse et à pores fins.

12. Utilisation d'un agent nettoyant capillaire et/ou cutané exemple de sulfate à base d'une combinaison de tensioactif particulièrement constituée
a. d'au moins un sel d'acide acylaminé de la formule (VI) susmentionnée,
b. d'au moins un alkylpolyglucoside,
c. d'au moins un tensioactif zwittérionique et/ou amphotère et
d. au moins un composé de la formule (VII), 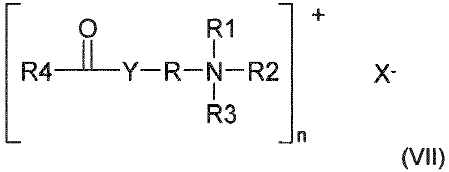 dans laquelle
• R4 est un long groupe carboné aliphatique ayant de 8 à 24 atomes de carbone,
• Y est un hétéroatome choisi parmi O, N ou S, dans le cas où Y=N l'atome d'azote porte encore un substituant -H ou -alkyle en C₁ à C₄,
• R est un groupe alkylène linéaire ou ramifié ayant de 1 à 10 atomes de carbone,
• R1, R2 et R3 sont indépendamment un groupe alkyle en C₁ à C₈, hydroxyalkyle en C₁ à C₈ ou benzyle, et
• X est un anion tel qu'un chlorure, un bromure, un méthosulfate, un éthosulfate, un tosylate, un acétate, un lactate, un phosphate ou un nitrate
pour améliorer le toucher, la capacité de peignage et la souplesse des fibres de kératine.
